# EUROPEAN PATENT APPLICATION

(11) **EP 4 656 652 A1**
(43) Date of publication of application: **03.12.2025**
(21) Application number: 24747430.7
(22) Date of filing: 23.01.2024
(51) Int. Cl.: C07K 14/46, A61P 29/00, A61P 25/28, A61K 38/00, A61K 38/26, A61K 38/17, A61P 25/00

(54) **NOVEL BRAIN DISEASE THERAPEUTIC AND USE THEREOF**

(30) Priority: 26.01.2023 KR 20230010421; 24.04.2023 KR 20230053102
(71) Applicant: Avixgen Inc., Seoul 08511 (KR)
(72) Inventor: YOU, Ji Chang, Seoul 06523 (KR); SUNG, Bo Kyung, Seoul 06253 (KR); BAEK, Yi Yong, Wonju-si Gangwon-do 26346 (KR)
(74) Representative: Weickmann & Weickmann PartmbB
(86) International application number: PCT/KR2024/001075
(87) International publication number: WO 2024/158194

(57) **Abstract**

The present invention relates to a novel brain disease therapeutic and a use thereof. A peptide or pharmaceutical composition according to the present invention can increase the level of delivery to a target site (in particular, inflamed areas of the brain) due to having a high blood-brain barrier penetration level, can have an increased half-life in the bloodstream, and can exhibit anti-inflammatory activity and treat or prevent brain diseases by reducing the level of inflammation.

## Description

### Technical Field

The present disclosure relates to a novel brain disease therapeutic and use thereof.

### Background Art

Neurodegenerative diseases such as Alzheimer's disease and Parkinson's disease predominantly affect the elderly population, and with the ongoing aging of society, the number of patients is exponentially increasing. In addition, early-onset forms of neurodegenerative diseases are also being reported with increasing frequency among younger individuals. As a result, there is growing interest in developing various therapies aimed at halting disease progression or restoring damaged brain tissues.

To improve or treat such neurodegenerative diseases, drug delivery methods targeting damaged brain tissue may be employed. However, to achieve this, the therapeutic agent must cross the blood-brain barrier (BBB). The BBB severely limits the transport of substances in the blood-including most macromolecules such as immunoglobulins (Ig), antibodies, complement proteins, and albumin-into brain tissue. Even drugs and small molecules have limited ability to cross the BBB, thus presenting a major obstacle to effective treatment.

Accordingly, there is a need for the development of novel formulations that can deliver biological substances effectively in vivo without inducing cytotoxicity. Recently, various alternatives have been proposed, and among them, cell-permeable peptides (CPPs) have attracted attention for their ability to improve the utilization of macromolecules-such as therapeutic proteins and genes-that have been difficult to use as drugs due to poor membrane permeability and short in vivo half-lives.

In light of this, the inventors of the present invention have conducted studies to develop a composition capable of improving blood-brain barrier permeability and exhibiting preventive or therapeutic effects against neurodegenerative brain diseases, thereby completing the present invention.

### DISCLOSURE

### Technical Problem

One aspect of the present invention is to provide a peptide represented by the amino acid sequence of SEQ ID NO: 1 or 2.

Another aspect of the present invention is to provide a pharmaceutical composition for anti-inflammatory use comprising the peptide, a method for treating an inflammatory disease comprising administering the peptide to a subject in need thereof, and a use of the peptide for the treatment or prevention of an inflammatory disease.

### Technical Solution

To achieve the above objects, one aspect of the present invention provides a peptide represented by the amino acid sequence of SEQ ID NO: 1 or 2.

In one embodiment, the peptide may suppress the expression of one or more of IL-1β, IL-6, Iba1, and TNFα, and increase the expression of Arg-1.

Another aspect of the present invention provides a pharmaceutical composition for anti-inflammatory use comprising the peptide.

In one embodiment, the inflammation may occur in the brain.

In another embodiment, the pharmaceutical composition may be for the treatment or prevention of a brain disease.

In another embodiment, the brain disease may be one or more selected from the group consisting of Alzheimer's disease (AD), Huntington's disease (HD), and Parkinson's disease (PD).

Still another aspect of the present invention provides a method for treating an inflammatory disease comprising administering the peptide to a subject, and a use of the peptide for the treatment or prevention of an inflammatory disease.

### Advantageous Effects

The peptide or pharmaceutical composition of the present invention exhibits high permeability through the blood-brain barrier (BBB), thereby enhancing delivery and accumulation at the site of action, particularly inflammatory regions within the brain. It may also prolong the peptide's half-life in blood circulation and improve inflammatory conditions, thereby enabling effective anti-inflammatory action as well as treatment or prevention of brain diseases.

### Brief Description of Drawings

FIG. 1 illustrates the enhancement of blood-brain barrier (BBB) permeability and plasma pharmacokinetics (PK) of exendin-4 by ACP. Specifically, FIG. 1A shows the results of interstitial fluid (ISF) analysis over time following administration of exendin-4, and ACP2-exendin-4 and ACP4-exendin-4, with doses adjusted to provide the same molar amount of exendin-4. FIG. 1B presents the pharmacokinetic (PK) parameters of exendin-4 in the plasma of rats to determine whether the time-dependent profiles observed in ISF were attributable to the in vivo distribution of exendin-4.
FIG. 2 shows the enhanced distribution of ACP-exendin-4 in different brain regions of rats.
FIG. 3 illustrates the in vitro anti-inflammatory effects of ACP-exendin-4.

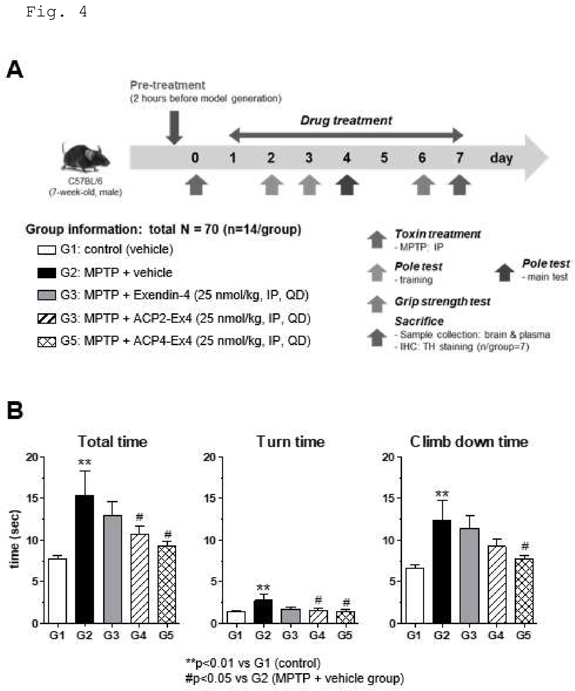

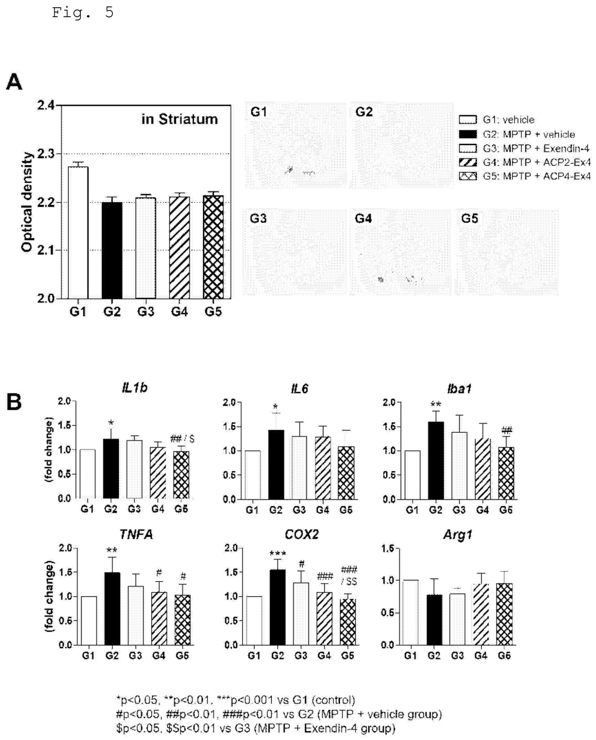

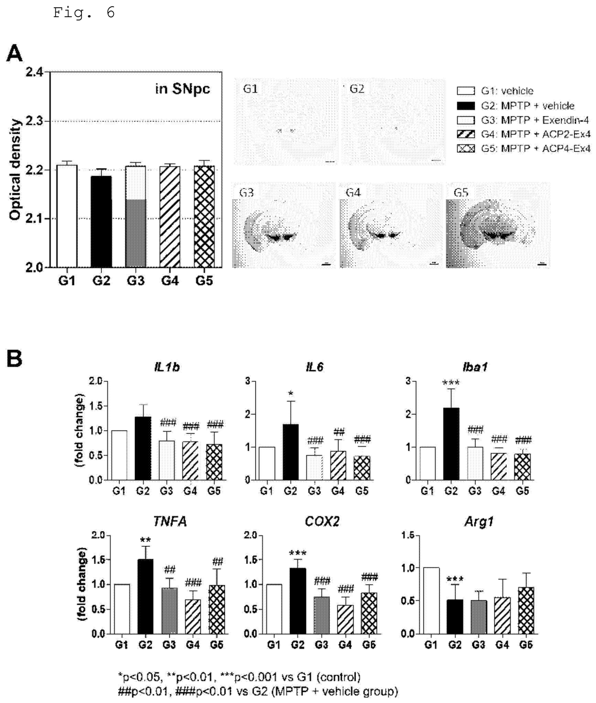

FIGS. 4 to 6 illustrate the experimental procedures and results intended to confirm the protective effects of ACP-exendin-4 against Parkinson's disease-like symptoms. Specifically, FIG. 4A shows the experimental scheme, and FIG. 4B illustrates the results of the pole test assessing improvements in motor function. FIGS. 5A and 6A show the reduction of tyrosine hydroxylase (TH) in the striatum and substantia nigra pars compacta(SNpc) of MPTP-administered groups compared to untreated controls. FIGS. 5B and 6B show the expression changes of pro-inflammatory cytokines (IL-1β, IL-6, Iba1, TNFα), the inflammatory mediator COX-2, and the anti-inflammatory marker Arg-1 in the striatum and SNpc, respectively.
FIG. 7 depicts cAMP production and β-arrestin-2 recruitment induced by ACP-exendin-4. Specifically, FIG. 7A shows cAMP generation, and FIG. 7B demonstrates β-arrestin-2 recruitment following ACP-exendin-4 treatment

### Best Mode

One aspect of the present invention provides a peptide represented by amino acid sequence No. 1 or 2.

Each of the amino acid sequences has a structure in which a cell-penetrating peptide (ACP2 or ACP4) is conjugated with Exendin-4.

**[Table 1]**

| Name | Sequence (Exendin-4 sequence is underlined) | Sequence No. |
|---|---|---|
| ACP2-exendin-4 | VKCFNCGKEGHTARNCRAPRKKGHGEGTFTSDLSKQMEEEAVRLFIEWLK NGGPSSGAPPPS | 1 |
| ACP4-exendin-4 | ARAPRKKGARAPRKKGHGEGTFTSDLSKQMEEEAVRLFIEWLKNGGPSSG APPPS | 2 |
| exendin-4 | HGEGTFTSDLSKQMEEEAVRLFIEWLKNGGPSSGAPPPS | 3 |
| ACP2 | VKCFNCGKEGHTARNCRAPRKKG | 4 |
| ACP4 | ARAPRKKGARAPRKKG | 5 |

As used herein, the term "cell-penetrating peptide (CPP)" refers to a cell membrane-permeable peptide consisting of a short peptide of about 10 to about 60 amino acids, which can move into a cell without damaging the cell membrane and can intracellularly deliver even DNAs or proteins that cannot pass through the cell membrane.

The exendin-4 (Exendin-4) is a 39-amino acid peptide produced by the salivary glands of the Gila monster (Heloderma suspectum). Exendin-4 acts as an agonist of the glucagon-like peptide-1 (GLP-1) receptor, but does not meaningfully activate the glucagon receptor. In 2005, Eli Lilly and Amylin Pharmaceuticals developed a synthetic version of exendin-4 known as Byetta^{®}, which was approved as a treatment for diabetes.

Conventionally, active ingredients such as exendin-4 have shown difficulty penetrating the blood-brain barrier (BBB), thus making it challenging for the therapeutic substance to reach its site of action. However, the peptide described herein is formed by conjugating exendin-4 with a cell-penetrating peptide such as ACP2 or ACP4, thereby enhancing the BBB permeability of the inventive peptide and improving its delivery and accumulation at inflamed brain regions. Additionally, such conjugation may increase the peptide's half-life in the bloodstream.

The peptide may inhibit the expression of one or more of IL-1β, IL-6, Iba1, and TNFα, while increasing the expression of Arg-1.

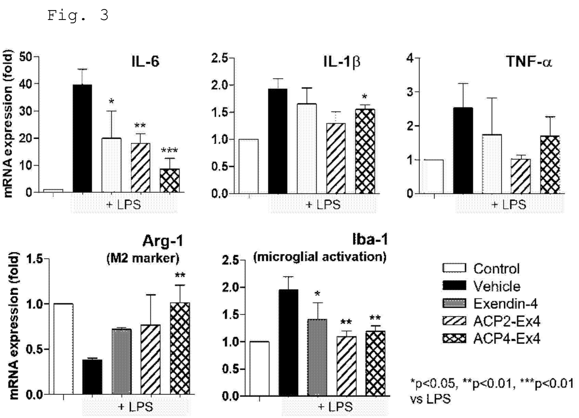

IL-1β, IL-6, Iba1, and TNFα are known as inflammatory markers. Accordingly, the inventive peptide may reduce the expression of the above-mentioned inflammatory markers, particularly in the brain, as compared to administration of exendin-4 alone. Arg-1 is a marker of M2 polarization in microglial cells, and the inventive peptide may increase the expression of this marker, in comparison to administration of exendin-4 (see FIGS. 3, 5, and 6).

As used herein, the term "conjugation" refers to the connection of a cell-penetrating peptide and a biologically or pharmaceutically active substance via covalent or noncovalent chemical or physical bonding.

Another aspect of the present invention provides a pharmaceutical composition for anti-inflammatory use comprising the peptide described above.

The pharmaceutical composition may include the aforementioned peptide as an active ingredient, either alone or in combination with known components.

Another aspect of the invention is that the inflammation may occur in the brain. Specifically, the pharmaceutical composition may be used for the treatment or prevention of neurological disorders, which may include one or more of Alzheimer's disease (AD), Huntington's disease (HD), and Parkinson's disease (PD).

In addition to the above-described fusion protein, the pharmaceutical composition of the present invention may further comprise a pharmaceutically acceptable carrier, as necessary.

Such pharmaceutically acceptable carriers are commonly used in the manufacture of pharmaceutical products and include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate, and mineral oil. The pharmaceutical composition of the present invention may also include additives such as lubricants, humectants, sweeteners, flavoring agents, emulsifiers, suspending agents, and preservatives.

The carrier may be included in the pharmaceutical composition of the present invention in an amount of approximately 1 wt% to approximately 99.99 wt%, preferably from approximately 90 wt% to approximately 99.99 wt%, based on the total weight of the composition. The additives may be included in an amount of approximately 0.1 wt% to approximately 20 wt%.

Meanwhile, the pharmaceutical composition of the present invention may be administered orally or non-orally, and may be applied topically directly to the skin.

The pharmaceutical composition of the present invention may be formulated using pharmaceutically acceptable carriers and/or excipients into a unit dosage form, or may be filled into a multi-dose container. In such cases, the dosage form may be a solution, suspension, or emulsion, or may include elixirs, extracts, powders, granules, tablets, hard capsules, soft capsules, lotions, or ointments.

The daily dosage of the pharmaceutical composition of the present invention is typically within a range of 0.001 to 150 mg/kg of body weight and may be administered in a single dose or divided doses. However, the dosage of the pharmaceutical composition of the present invention should be determined in consideration of several relevant factors including the administration route, age, sex, body weight, and disease severity of the patient, and therefore, the above-mentioned dosage should not be construed as limiting the scope of the present invention in any respect.

As previously described, active substances such as exendin-4 conventionally show poor permeability across the blood-brain barrier (BBB), making it difficult to reach the target site of action. However, the peptide described herein is formed by conjugating exendin-4 with a cell-penetrating peptide, such as ACP2 or ACP4, thereby increasing BBB permeability and enhancing delivery to and accumulation at the target site (particularly, the inflamed regions in the brain). This conjugation may also extend the peptide's half-life in the bloodstream. Additionally, compared to administration of exendin-4 alone, the peptide of the present invention may inhibit the expression of one or more of IL-1β, IL-6, Iba1, and TNFα, and may increase the expression of Arg-1. Through these effects, the peptide or the pharmaceutical composition of the present invention may suppress inflammation in brain tissue and may be used to treat or prevent brain diseases such as Alzheimer's disease (AD), Huntington's disease (HD), and Parkinson's disease (PD).

The present invention also provides a method for treating inflammatory diseases comprising administering the peptide to a subject suffering from an inflammatory disorder, and further provides the use of the peptide for treating or preventing inflammatory diseases.

### Mode for Invention

Hereinafter, the present disclosure will be described in more detail with reference to one or more examples. However, these examples are to illustrate the present disclosure, and the scope of the present disclosure is not limited to these examples.

### Example 1: Experimental Method

### Intravenous Pharmacokinetic Study Using SD Rats

Exendin-4 (1 mg/kg), ACP2-Exendin-4 (1.6 mg/kg), and ACP4-Exendin-4 (1.4 mg/kg), each dissolved in PBS, were administered intravenously to SD rats. Whole blood was collected at pre-dose (0), and at 1, 5, 15, 30, 45 minutes and 1, 2, 4, and 6 hours post-dose. Plasma was separated and stored in a deep freezer at approximately -70°C until analysis. Plasma concentrations of the compounds were quantified using LC-MS/MS. The Exendin-4 (SEQ ID NO: 3), ACP2-Exendin-4 (SEQ ID NO: 1), and ACP4-Exendin-4 (SEQ ID NO: 2) used in this study were synthesized by Chempeptide Limited with a purity of ≥ 98%.

### Cell Lines and Culture Conditions

All cells were cultured at 37°C in a humidified atmosphere containing 5% CO₂. BV2 cells were maintained in DMEM supplemented with 10% FBS. PathHunter^{®} CHO-K1 GLP1R bioassay cells were cultured in the medium provided with the kit.

### Real-Time PCR

BV2 cells were seeded into 12-well plates and incubated overnight, followed by treatment with test compounds at varying concentrations. After 1 hour of incubation, lipopolysaccharide (LPS) was added to a final concentration of 1 µg/mL, and cells were incubated for an additional 6 hours. Total RNA was extracted using TRI reagent. cDNA synthesis was followed by real-time PCR to examine the expression levels of inflammation-related genes.

### β-Arrestin-2 Recruitment Assay (PathHunter^{®} CHO-K1 GLP1R Bioassay)

The β-arrestin-2 recruitment assay was performed using PathHunter^{®} CHO-K1 GLP1R bioassay cells. After cell seeding, test compounds were applied at concentrations ranging from 25.6 pM to 10 µM. Following 30 minutes of incubation, Detection Reagent 1 was added to each well and incubated for 15 minutes at room temperature in the dark. Subsequently, Detection Reagent 2 was added and the plates were incubated for 1 hour at room temperature in the dark. Luminescence was then measured.

### cAMP Assay

Intracellular cAMP levels were measured using the cAMP Parameter Assay Kit (R&D Systems). BV2 cells were seeded into 6-well plates and incubated overnight. Test compounds were administered at a final concentration of 100 nM and incubated for 15 minutes. Cells were washed three times with cold PBS and harvested using a cell scraper. After centrifugation, the supernatant was discarded and the cell pellet was resuspended in 300 µL of Cell Lysis Buffer 5. Following two freeze/thaw cycles, cell lysates were prepared by centrifugation. The assay was conducted according to the kit instructions. After adding 200 µL of Substrate Solution, samples were incubated for 30 minutes at room temperature in the dark, followed by addition of 100 µL of Stop Solution. Absorbance was measured at 450 nm using a microplate reader and cAMP concentrations were calculated according to the kit protocol.

### Blood-Brain Barrier (BBB) Penetration Assessment of ACP-Exendin-4 Using Microdialysis

To evaluate whether ACP enhances the BBB permeability of Exendin-4 in interstitial fluid (ISF) of the brain, microdialysis was performed. Rats were secured in a stereotaxic frame, and a hole was drilled into the skull at a coordinate corresponding to the striatum (based on the bregma) using stereotaxic surgery. A microdialysis guide cannula was implanted into the hole. After recovery from surgery, a microdialysis probe was inserted into the guide cannula. The rat was placed in a microdialysis cage, and tubing was connected to a syringe pump and fraction collector, with the system stabilized to ensure steady flow of dialysate. Exendin-4 (10 mg/kg), ACP2-Exendin-4 (16 mg/kg), and ACP4-Exendin-4 (14 mg/kg) were administered via tail vein injection. Microdialysis sampling of ISF was conducted at multiple time points. The concentrations of Exendin-4, ACP2-Exendin-4, and ACP4-Exendin-4 in the collected ISF samples were analyzed using LC-MS/MS.

### Example 2: Experimental Results

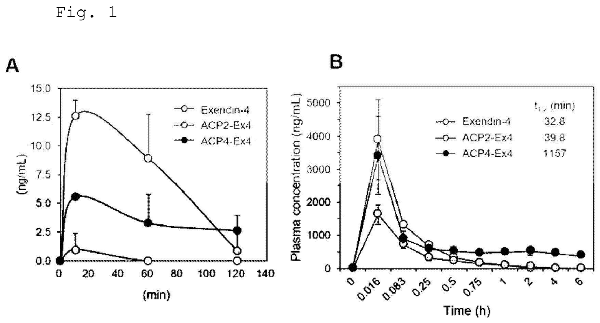

### Enhanced BBB Penetration and Plasma Pharmacokinetic Profile of Exendin-4 via ACP Conjugation (Fig. 1)

After tail vein injection of 10 mg/kg exendin-4 or doses of ACP2-exendin-4 and ACP4-exendin-4 adjusted to deliver equimolar amounts of exendin-4 into rats, time-dependent analysis of ISF revealed that, at 10 minutes post-administration, ACP2- and ACP4-conjugated exendin-4 (ACP2-exendin-4: 12.63 ± 1.35 ng/mL; ACP4-exendin-4: 5.56 ± 0.13 ng/mL) exhibited concentrations 6-12 times higher than unconjugated exendin-4 (0.99 ± 1.38 ng/mL) (Fig. 1A). While exendin-4 was detected at low levels in ISF only at 10 minutes post-administration, ACP2- and ACP4-exendin-4 remained detectable up to 1 hour, with ACP4-exendin-4 showing sustained presence beyond that time point (Fig. 1A).

To determine whether differences in ISF concentration profiles were attributable to systemic distribution, pharmacokinetic parameters were evaluated in rat plasma. Following IV injection of exendin-4 (1 mg/kg), ACP2- or ACP4-exendin-4, at doses adjusted to provide an equivalent molar amount of exendin-4, peak plasma concentrations were observed at 1 minute post-dose and then declined over time (Fig. 1B).

Among the tested compounds, ACP4-exendin-4 displayed a significantly greater area under the concentration-time curve from time zero to the last measurable concentration (AUCₗₐₛₜ) and extended plasma half-life compared to both exendin-4 and ACP2-exendin-4. Furthermore, the ISF profile of ACP4-exendin-4 closely mirrored its plasma ACP4-exendin-4 profile, indicating systemic-CNS exposure correlation. These findings suggest that conjugation of exendin-4 with ACP2 or ACP4 enhances not only BBB permeability but also in vivo stability, supporting the potential of these conjugates as effective drug candidates for the treatment of CNS disorders.

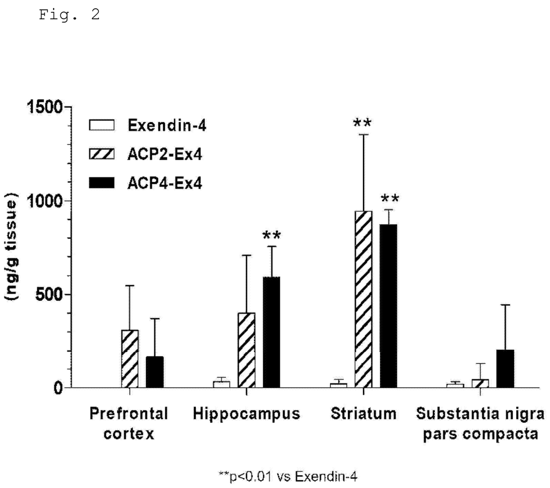

### Confirmation of Region-Specific Brain Distribution of ACP-Exendin-4 in Rats (Fig. 2)

In the context of developing effective therapeutics for brain disorders, ensuring sufficient and sustained drug exposure at target brain sites is a critical objective in central nervous system (CNS) drug development. Accordingly, the regional brain distribution of exendin-4 and ACP-conjugated exendin-4 (ACP2-exendin-4 and ACP4-exendin-4) was investigated. Rats were administered exendin-4 (10 mg/kg), ACP2-exendin-4 or ACP4-exendin-4, with doses normalized to the molar amount of exendin-4 via tail vein injection. Ten minutes post-dose, animals were sacrificed, and brain tissues were harvested. The concentrations of test substances in individual brain regions were then quantified via LC-MS/MS.

Four brain regions-hippocampus, prefrontal cortex, striatum, and substantia nigra pars compacta (SNpc)-were selected for analysis based on their known susceptibility to neurodegeneration in diseases such as Alzheimer's disease (AD), Huntington's disease (HD), and Parkinson's disease (PD). In the prefrontal cortex, exendin-4 was not detected. In contrast, ACP2-exendin-4 and ACP4-exendin-4 were observed at concentrations of 307 ng/mL and 161 ng/mL, respectively.

Parkinson's disease is characterized by dopaminergic neuronal degeneration in the SNpc, leading to decreased dopamine levels in the striatum; these two regions are well established as primary pathological sites in PD. In these areas, unmodified exendin-4 showed low distribution levels (22-24 ng/mL). However, delivery of ACP-conjugated exendin-4 was enhanced significantly-ranging from at least 2.3-fold to over 40-fold compared to exendin-4. Moreover, ACP-conjugated exendin-4 was also effectively delivered to the hippocampus, a principal site of pathology in AD. These results indicate that ACP conjugation enables differential and enhanced distribution to key brain regions and suggest that ACP-exendin-4 may exert superior therapeutic effects over exendin-4 in treating neurodegenerative diseases such as PD and AD.

### In Vitro Anti-Inflammatory Effects of ACP-Exendin-4 (Fig. 3)

The anti-inflammatory effects of the test compounds were evaluated using BV2 microglial cells, which are known to participate in neuroinflammatory responses in the brain. Neuroinflammation is a critical and unavoidable pathological process associated with all types of injury and disease affecting the central nervous system and represents a hallmark of the neurodegenerative disease environment. Microglia can polarize into either the pro-inflammatory M1 phenotype, which exacerbates neurotoxicity, or the anti-inflammatory M2 phenotype, which contributes to neuroprotection, depending on microenvironmental stimuli. It has been reported that lipopolysaccharide (LPS) induces polarization of microglia toward the M1 phenotype. When BV2 cells were stimulated with LPS to induce an inflammatory response, upregulation of pro-inflammatory cytokines-IL-6, IL-1β, and TNF-α-as well as of Iba1, a marker of microglial activation, was observed. Pretreatment with the test compounds resulted in a reduction in the expression levels of IL-6, IL-1β, TNF-α, and Iba1 compared to the LPS-treated control group (Fig. 3). In particular, pretreatment with exendin-4 and ACP-exendin-4 significantly suppressed the expression of IL-6 and Iba1, with ACP-exendin-4 demonstrating a greater inhibitory effect than exendin-4. Although the anti-inflammatory effects of ACP-exendin-4 on IL-1β and TNF-α expression were confirmed, the reductions did not reach statistical significance (Fig. 3).

Arg-1, known as a marker for M2 polarization of microglia and functioning as an anti-inflammatory factor, has been reported to be downregulated by LPS treatment. Consistent with previous findings, Arg-1 expression was decreased in BV2 cells stimulated with LPS in the present study. The LPS-induced reduction in Arg-1 mRNA expression was restored by pretreatment with exendin-4 and ACP-exendin-4, and ACP-exendin-4 exhibited a greater restorative effect than exendin-4. These results indicate that ACP-exendin-4 possesses superior anti-inflammatory activity compared to exendin-4.

### Protective Effects of ACP-Exendin-4 on Parkinson's Disease-Like Symptoms (Figs. 4-6)

### (1) Pole Test

To evaluate the effect of ACP-conjugated exendin-4 on Parkinson's disease (PD)-like symptoms, studies were conducted using an MPTP-induced PD mouse model (Fig. 4A). Motor performance was assessed via the pole test, in which the "time to turn," "climb down time," and "total time to descend" were measured. In the MPTP-treated group (G2), all time measures were significantly increased. In contrast, groups treated with ACP2-exendin-4 (G4) or ACP4-exendin-4 (G5) exhibited a significant reduction in both total descent time and turn time compared to the MPTP-only group (Fig. 4B).

### (2) Tyrosine Hydroxylase (TH) Immunostaining

To investigate the neuroprotective effects of ACP-exendin-4 against MPTP-induced dopaminergic neuron loss, the brains of treated mice were dissected, and the striatum and substantia nigra were subjected to immunohistochemical staining to evaluate the expression of tyrosine hydroxylase (TH).

TH is a rate-limiting enzyme in the synthesis of dopamine and epinephrine from tyrosine. In mammals, TH is expressed specifically in the hypothalamus, midbrain, brainstem, and olfactory bulb, and is a marker with high tissue specificity. Dysregulation of TH expression following damage or impaired differentiation of midbrain dopaminergic neurons is a known pathological feature of PD. In the MPTP group, a marked decrease in TH expression was observed in both the striatum and substantia nigra compared to the untreated control group. However, the groups administered with test compounds showed no statistically significant differences in TH expression relative to the MPTP-only group (Figs. 5A and 6A).

### (3) Suppression of Pro-Inflammatory Cytokine Expression

To assess the anti-inflammatory effect of ACP-exendin-4, qPCR was performed using brain tissues-specifically the striatum and substantia nigra pars compacta (SNpc)-obtained from the MPTP-induced PD mouse model. In the striatum, the MPTP-treated group (G2) exhibited the highest expression levels of inflammatory cytokines (IL-1β, IL-6, Iba1, TNFα) and the inflammatory mediator COX-2. It was confirmed that the groups treated with exendin-4 or ACP-exendin-4 showed lower expression levels of the inflammatory cytokines and COX-2 compared to the MPTP-treated group. When comparing efficacy, the result revealed the following order: ACP4-exendin-4>ACP2-exendin-4>exendin-4. Furthermore, with respect to IL-1β and COX-2, ACP4-exendin-4 showed a statistically significant difference compared to exendin-4, indicating that ACP4-exendin-4 exerts a greater anti-inflammatory effect than exendin-4 (Fig. 5B). Also in the SNpc region, the MPTP-treated group showed the strongest inflammatory response, which was confirmed to be suppressed by administration of the test compounds, consistent with the results in the striatum (Fig. 6B). Although the order of anti-inflammatory efficacy (ACP4-exendin-4 > ACP2-exendin-4 > exendin-4) was not clearly confirmed in the SNpc results, it was nonetheless confirmed that ACP-conjugated exendin-4 possesses stronger anti-inflammatory effects than exendin-4. As for Arg-1, an anti-inflammatory marker, its expression was confirmed to be reduced by MPTP induction in both the striatum and SNpc, while its level was restored by administration of the test compounds. Although the difference was not statistically significant, compared to exendin-4 (G3), both ACP2-exendin-4 (G4) and ACP4-exendin-4 (G5) demonstrated a greater ability to recover Arg-1 expression, indicating that ACP-exendin-4 has superior anti-inflammatory activity compared to exendin-4 (Figs. 5B and 6B).

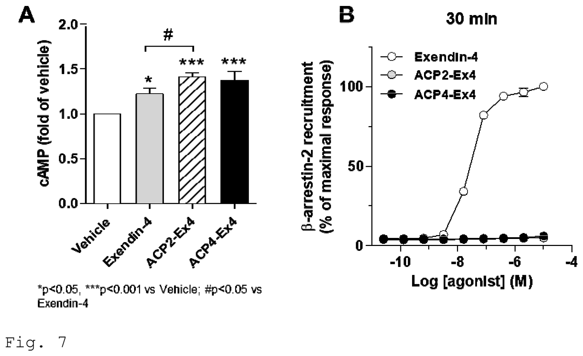

### (4) cAMP Production and β-Arrestin-2 Recruitment by ACP-Exendin-4 (Fig. 7)

To elucidate the mechanism of action of ACP-exendin-4, it was first evaluated whether exendin-4 conjugated with ACP mediates signaling through GLP-1R, the receptor for exendin-4, by measuring cAMP production. When BV2 cells were treated with the test compounds, it was confirmed that the amount of cAMP produced significantly increased compared to the vehicle control. Moreover, cAMP production induced by ACP2-exendin-4 was significantly higher than that by exendin-4 (Fig. 7A). Exendin-4 is taken up into cells via GLP-1R, during which process cAMP is generated. Therefore, these results suggest that ACP-exendin-4 is taken up via GLP-1R.

To further examine the activation mechanism of GLP-1R by ACP-exendin-4, the PathHunter^{®} bioassay detection kit was used to detect β-arrestin signaling, one of the downstream pathways of GLP-1R. Exendin-4 induced β-arrestin-2 recruitment in a concentration-dependent manner, whereas ACP-conjugated exendin-4 showed little or no β-arrestin-2 recruitment (Fig. 7B). These results suggest that ACP-conjugated exendin-4 is taken up via GLP-1R but acts as a G protein-biased agonist that does not activate β-arrestin signaling.

Those skilled in the art to which the present invention pertains will appreciate that the present disclosure may be embodied in modified forms without departing from the essential characteristics of the present disclosure. Therefore, it should be understood that the disclosed embodiments are illustrative in all aspects and are not restrictive. The scope of the present disclosure is defined by the claims rather than the foregoing description, and all differences within the scope equivalent to the claims should be construed as falling within the scope of the present invention.

## Claims

1. A peptide represented by an amino acid sequence of SEQ ID NO: 1 or 2.

2. The peptide according to claim 1, wherein the peptide suppresses expression of one or more selected from the group consisting of IL-1β, IL-6, Iba1, and TNFα, and increases expression of Arg-1.

3. A pharmaceutical composition for anti-inflammatory use, comprising the peptide according to claim 1.

4. The pharmaceutical composition according to claim 3, wherein the inflammation occurs in the brain.

5. The pharmaceutical composition according to claim 3, wherein the pharmaceutical composition is for the treatment or prevention of a brain disease.

6. The pharmaceutical composition according to claim 5, wherein the brain disease is one or more selected from the group consisting of Alzheimer's disease (AD), Huntington's disease (HD), and Parkinson's disease (PD).

7. A method for treating an inflammatory disease, comprising administering the peptide according to claim 1 to a subject in need thereof.

8. Use of the peptide according to claim 1 for the treatment or prevention of an inflammatory disease.
